# EUROPEAN PATENT APPLICATION

(11) **EP 4 552 656 A1**
(43) Date of publication of application: **14.05.2025**
(21) Application number: 23834840.3
(22) Date of filing: 04.07.2023
(51) Int. Cl.: A61K 51/04, A61K 51/12, C07D 403/12, C07B 59/00, A61K 101/02

(54) **LIQUID COMPOSITION OF COMPOUND I, PREPARATION METHOD THEREFOR, AND APPLICATION THEREOF**

(30) Priority: 04.07.2022 CN 202210778537
(71) Applicant: Sinotau Pharmaceutical Group, Beijing 100176 (CN)
(72) Inventor: WANG, Yue, Beijing 100176 (CN); ZHANG, Ying, Beijing 100176 (CN); ZHANG, Aili, Beijing 100176 (CN); XU, Xinsheng, Beijing 100176 (CN)
(74) Representative: Lavoix
(86) International application number: PCT/CN2023/105706
(87) International publication number: WO 2024/008073

(57) **Abstract**

The present application provides a liquid composition of Compound I, a preparation method therefor, and an application thereof, wherein same is selected from one or more among vitamin C, sodium vitamin C, and gentisic acid. By optimizing process parameters and technological process, the method of the present application can be applied to large-scale production, and the product prepared has high radiochemical purity, high activity and concentration, good product stability, high and stable yield or productivity.

## Description

### TECHNICAL FIELD

The present application belongs to the field of chemical pharmaceutical technology, particularly relates to a liquid composition of Compound I, a preparation method and use thereof.

### BACKGROUND

Myocardial perfusion imaging has been used for non-invasive examination of heart disease since the 1970s. Its great diagnostic value has been widely accepted worldwide and has become one of the most important imaging methods for the diagnosis, efficacy evaluation and prognosis of coronary heart disease. Myocardial perfusion single-photon emission computed tomography (SPECT) imaging technology is the primary non-invasive perfusion imaging method currently used in clinical detection of coronary heart disease. However, compared with SPECT, positron emission tomography (PET) imaging has higher spatial and temporal resolution, can effectively reduce tissue attenuation, and can achieve absolute quantification of coronary blood flow using standard tissue attenuation correction methods. In addition, the short half-life of positron nuclides can effectively reduce the radiation dose around the target tissue, and the short half-life can also shorten the interval between resting and motion imaging. Commonly used myocardial perfusion PET imaging agents include: ¹⁵O-H₂O, ¹³N-NH₃·H₂O and ⁸²Rb, etc. However, the half-lives of the above-mentioned imaging agents are relatively short, and their clinical applications are still greatly limited. Compared with other positron-emitting nuclides, ¹⁸F has a longer half-life (t_{1/2} = 109.8 min); it has lower positron energy (average energy 249.8 keV), and causes less radiation damage to normal tissues; its van der Waals radius (1.35) is similar to that of hydrogen (1.2), and will not affect the biological activity of the labeled compound. Therefore, developing new fluorine-18 labeled myocardial perfusion imaging agent holds significant practical importance.

Compound I, whose chemical name is 2-tert-butyl-4-chloro-5-((3-((4-((2-(2-fluoro[¹⁸F]ethoxy)ethoxy)methyl)-1*H*-1,2,3-tria zol-1-yl)methyl)benzyl)oxy)pyridazin-3(2*H*)-one, comprises the radioactive nuclide ¹⁸F and can be used for positron emission tomography (PET) imaging. With the characteristic of minimal chemical content and the inability to isolate pure products, the prepared products mostly exist in solution form.

### SUMMARY

In the prior art, the preparation of Compound I has the following problems: high concentration of ¹⁸F ions added but low yield of the final product. In addition, the radiochemical purity of the prepared Compound I is not high, especially the radiochemical purity decreases significantly after being left for a period of time, and the stability cannot meet the application requirements.

The purpose of the present application is to provide a large batch of liquid composition of Compound I that meets the quality requirements: high radiochemical purity, high activity concentration, good product stability, and high and stable yield or production rate.

The purpose of the present application is to provide a method for automated preparation of Compound I liquid compositions in large-scale to meet clinical medication needs and design a continuous and stable production process. The technical route provided in this application can meet the requirements of high initial ¹⁸F activity labeling rate, and optimize the amount of precursor used in the reaction, reaction time and purification HPLC conditions, thereby improving the reaction yield and the radiochemical purity and stability of Compound I.

The technical solutions of this application are as follows:
1. A method for preparing a liquid composition of Compound I, comprising the following steps:
   purifying the crude product comprising Compound I by high-performance liquid chromatography;
   wherein the mobile phase used in the purification step by high-performance liquid chromatography comprises ethanol and water;
   and the Compound I is 2-tert-butyl-4-chloro-5-((3-((4-((2-(2-fluoro[¹⁸F]ethox y)ethoxy)methyl)-1*H*-1,2,3-triazol-1-yl)methyl)benzyl)oxy)pyridazin-3(2*H*)-one;
   the mobile phase further comprises one or more of sodium vitamin C, vitamin C and gentisic acid.
2. The method according to item 1, wherein in the purification step by high-performance liquid chromatography, the ethanol is 0.2-2 volume parts relative to 1 volume part of the water in the mobile phase.
3. The method according to item 2, wherein the ethanol is 0.4-1 volume part relative to 1 volume part of water in the mobile phase.
4. The method according to item 1, wherein in the purification step by high-performance liquid chromatography, the addition amount of sodium vitamin C is 0-20 mg/mL, preferably 0.2-10 mg/mL.
5. The method according to item 1, wherein in the purification step by high-performance liquid chromatography, the addition amount of vitamin C is 0-10 mg/mL, preferably 0.1-5 mg/mL.
6. The preparation method according to item 1, wherein in the purification step by high-performance liquid chromatography, the addition amount of gentisic acid is 0-10 mg/mL, preferably 0.1-5 mg/mL.
7. The method according to item 1, wherein the chromatographic column used in the purification step by high-performance liquid chromatography is a silica gel column, preferably a reverse phase C18 silica gel chromatographic column, and more preferably an XBridge BEH C18 OBD Prep column;
   preferably, the purification is carried out by isocratic elution, and the elution flow rate of the mobile phase is 3-6 mL/min.
8. The method according to item 1, wherein the method further comprises a step of nucleophilic substitution reaction before the purification step by high-performance liquid chromatography;
   in the nucleophilic substitution reaction: the activated ¹⁸F ion is mixed with a solution comprising a Compound I precursor for the nucleophilic substitution reaction to generate a crude product comprising Compound I;
   and the Compound I precursor is methyl 2-(2-((1-(3-(((1-(tert-butyl)-5-chloro-6-oxo-1,6-dihydropyridazin-4-yl)oxy)methyl)be nzyl)-1*H*-1,2,3-triazol-4-yl)methoxy)ethoxy)ethyl-4-toluenesulphonate.
9. The method according to item 8, wherein in the nucleophilic substitution reaction, the reaction solvent is a non-protonic polar solvent, the ratio of the amount of Compound I precursor to the activity of ¹⁸F ion is in the range of (0.5-8):1; the reaction temperature is 90-140°C, the reaction time is 5-60min, and the reaction is in a sealed space;
   the unit of the amount of Compound I precursor is mg, and the unit of the activity of ¹⁸F ion is Ci.
10. The method according to item 9, wherein the non-protonic polar solvent is one or more of acetonitrile, dimethyl sulfoxide, N,N-dimethylformamide, and 2-methyl-2-butanol.
11. The method according to item 8, wherein the method further comprises a step for preparing ¹⁸F ion before the step of nucleophilic substitution reaction; and the step for preparing ¹⁸F ion further comprises a step for preparing a ¹⁸F ion solution, a step for enriching and eluting ¹⁸F ion, and a step for activating ¹⁸F ion; more preferably,
   in the step for preparing a ¹⁸F ion solution, the ¹⁸F ion solution is prepared by accelerator;
   in the step for enriching ¹⁸F ion, the ¹⁸F ion solution prepared above is enriched by an anion exchange cartridge;
   in the step for eluting ¹⁸F ion, ¹⁸F ions are eluted by eluting with a catalyst solution of cryptand and alkali metal salt catalyst;
   in the step for activating ¹⁸F ion: the solvent is blown dry by program-controlled temperature, nitrogen or other inert gas, and the ¹⁸F ions are activated to obtain the activated ¹⁸F ions.
12. The method according to item 11, wherein in the step for preparing a ¹⁸F ion solution, the water comprising ¹⁸O is transferred to the accelerator target site, and the accelerator is initiated to generate a proton beam to bombard the water comprising ¹⁸O to produce a solution comprising ¹⁸F ions; and the initial activity of the ¹⁸F is 0.045Ci-11Ci; preferably, the initial activity of the ¹⁸F is 3.15Ci-11Ci.
13. The method according to item 11, wherein in the step for enriching ¹⁸F ion, the anion exchange column is a tetraalkylammonium salt anion exchange column.
14. The method according to item 11, wherein in the step for eluting ¹⁸F ion, the amount of cryptand is 5-40 mg and the amount of alkali metal salt is 1.5-20 mg in the catalyst solution of cryptand and alkali metal salt;
   preferably, the cryptand is 4,7,13,16,21,24-hexaoxa-1,10-diazabicyclo[8,8,8]hexacosane (Kryptofix-2.2.2, amino polyether); the alkali metal salt is one or more of K₂CO₃, Na₂CO₃, Cs₂CO₃, KHCO₃, and NaHCO₃;
   further preferably, the catalyst solution is selected from a mixed solvent system of acetonitrile and water, wherein the volume ratio of acetonitrile to water is (0.2-10):1.
15. The method according to item 11, wherein in the step for activating 18F ion, the activation temperature is 80-130°C;
   temperature controlling by program comprises the following steps: evaporating for 60-120s under the condition of 100-120°C, positive pressure of 50-200mbar, and vacuum pressure -20~-60mbar; evaporating for 150-200s 1 under the condition of 20-130°C, positive pressure of 50-200mbar, and vacuum pressure -20~-60mbar; evaporating for 10-30s under the condition of 120-130°C, positive pressure of 50-200mbar, and vacuum pressure of -60~-100mbar,; evaporating for 80-120s under the condition of 100-120°C, positive pressure of 800-1200mbar, and vacuum pressure of -800~-1000mbar; evaporating for 100-120s under the condition of 80-100°C, positive pressure of 400-600mbar, and vacuum pressure of -800 ∼ -1000mbar; and evaporating for 10-20s under the condition of 80-100°C, positive pressure of 600-900mbar, and vacuum pressure of -800~-1000mbar.
16. Use of the Compound I liquid composition prepared by the method according to any of the items 1-15 in a myocardial perfusion PET imaging agent.

In addition, the present application provides a liquid composition comprising Compound I, and the specific scheme is as follows:
1. A liquid composition of Compound I, wherein it is one or more selected from the group consisting of vitamin C, sodium vitamin C, and gentisic acid;
   the Compound I is 2-tert-butyl-4-chloro-5-((3-((4-((2-(2-fluoro[¹⁸F]ethoxy)ethoxy)methyl)-1*H*-1,2,3-tria zol-1-yl)methyl)benzyl)oxy)pyridazin-3(2*H*)-one.
2. The liquid composition according to item 1, wherein the ratio of concentration of vitamin C (mg/mL) to activity concentration of Compound I (mCi/mL) is 0.02-0.2.
3. The liquid composition according to item 2, wherein the ratio of concentration of vitamin C (mg/mL) to activity concentration of Compound I (mCi/mL) is 0.04-0.15.
4. The liquid composition according to item 1, wherein the ratio of concentration of gentisic acid (mg/mL) to activity concentration of Compound I (mCi/mL) is 0.02-0.2.
5. The liquid composition according to item 4, wherein the ratio of concentration of gentisic acid (mg/mL) to activity concentration of Compound I (mCi/mL) is 0.04-0.15.
6. The liquid composition according to item 1, wherein the ratio of concentration of sodium vitamin C (mg/mL) to activity concentration of Compound I (mCi/mL) is 0.04-1.
7. The liquid composition according to item 6, wherein the ratio of concentration of sodium vitamin C (mg/mL) to activity concentration of Compound I (mCi/mL) is 0.1-1.
8. The liquid composition according to item 1, wherein the sodium vitamin C is 2-10 parts by weight relative to 1 part by weight of vitamin C.
9. The liquid composition according to item 8, wherein the sodium vitamin C is 3-10 parts by weight relative to 1 part by weight of vitamin C.
10. The liquid composition according to item 1, wherein the gentisic acid is 0.1-5 parts by weight relative to 1 part by weight of vitamin C.
11. The liquid composition according to item 10, wherein the gentisic acid is 0.1-3 parts by weight relative to 1 part by weight of vitamin C.
12. The liquid composition according to item 1, the liquid composition formula further comprises an aqueous solution of polyethylene glycol and/or ethanol.
13. The liquid composition according to item 12, wherein the concentration of polyethylene glycol is 0.1-0.3 g/mL, preferably 0.1-0.2 g/mL.
14. The liquid composition according to item 12, wherein the polyethylene glycol is polyethylene glycol 400.
15. The liquid composition according to item 12, wherein the ethanol is 5-20 mL relative to 100 mL of water.
16. A method for preparing the liquid composition according to any one of items 1 to 15, wherein the method comprises: dissolving one or more of vitamin C, sodium vitamin C and gentisic acid in water and stirring to form a solution, and mixing Compound I collected with the solution to obtain the liquid composition of Compound I.
17. The method according to item 16, wherein the method comprises: dissolving polyethylene glycols and/or ethanol, and one or more of vitamin C, sodium vitamin C and gentisic acid in water and stirring to form a solution, and mixing the Compound I collected with the solution to obtain the liquid composition of Compound I.
18. Use of the liquid composition of Compound I according to any one of items 1 to 15 or the liquid composition of Compound I prepared by the method according to item 16 or 17 in preparation of a myocardial perfusion PET imaging agent.
19. A method for performing myocardial perfusion on a subject, comprising administering to the subject the liquid composition of Compound I according to any one of items 1-9, or the liquid composition of Compound I prepared by the method according to item 10 or 11.

In addition, the present application provides a reagent kit for automated preparation of a liquid composition of Compound I, wherein the reagent kit comprises a reaction flask and a prescription bottle;
the reaction flask includes reaction flask R1, reaction flask R2, and reaction flask R3; wherein the reaction flask R1 is used to hold the eluent for eluting ¹⁸F ion; the reaction flask R2 is used to hold a solution of Compound I precursor for the ¹⁸F ion nucleophilic substitution reaction; the reaction flask R3 is used to hold reagents for diluting the crude products and rinsing the reaction system;
the prescription bottle includes prescription bottle P1 and prescription bottle P2; wherein the prescription bottle P1 is used to transfer the reaction products of ¹⁸F ion nucleophilic substitution reaction; the prescription bottle P2 is used to hold substances used to stabilize the reaction products of ¹⁸F ion nucleophilic substitution reaction.

Furthermore, the reaction flask R1 comprises amino polyether, potassium carbonate, water for injection, and acetonitrile; preferably, the concentration of the amino polyether is 5-40mg/mL, the concentration of potassium carbonate is 1.5-20mg/mL, and the volume ratio of acetonitrile to water is (0.25-19): 1;
alternatively, the reaction flask R2 comprises an acetonitrile solution of the Compound I precursor for ¹⁸F ion nucleophilic substitution reaction; preferably, the concentration of Compound I precursor in the acetonitrile solution is 1-10 mg/mL;
alternatively, the reaction flask R3 comprises anhydrous ethanol.

Furthermore, wherein the prescription bottle P1 comprises polyethylene glycol; preferably, the concentration of polyethylene glycol is 0.05-0.3 g/mL;
alternatively, the prescription bottle P2 comprises one or more of vitamin C, sodium vitamin C, and gentisic acid for stabilizing the reaction products of the ¹⁸F ion nucleophilic substitution reaction; preferably, the concentration of vitamin C is 2-16mg/mL; the concentration of sodium vitamin C is 15-40mg/mL, and the concentration of gentisic acid is 2-16mg/mL.

Compared with the prior art, the beneficial effects of the present application are:
(1) The present application optimizes the experimental process scheme, changes the amount of the Compound I precursor, shortens the reaction time required to achieve the same labeling rate, increases the radioactivity of the initial ¹⁸F ions, increases the labeling rate, and thus increases the yield. The process parameters and process flow are clear and specific, which can be applied to large-scale activity production and meet the automation requirements.
(2) In the optimized technical solution of the present application, a radiation-resistant solvent is used in the mobile phase of the purification process to avoid product loss due to radiolysis during the purification and formulation processes, resulting in higher yield. In addition, the present application uses an anti-radiation decomposition agent during the formulation process to ensure the stability of the product.
(3) This application optimizes the purification process by removing the C18 column and using an ethanol/water system as the mobile phase instead of an acetonitrile/water system, these measures simplify the time and improve the radiochemical purity and stability of the product.
(4) The reagent kit of this application brings convenience, cost savings, and precise control to the automated preparation of compound I liquid composition.
(5) In the prior art, samples are generally prepared temporarily, and each batch needs to be inspected by personnel before release, which leads to high workload, low production efficiency, and high costs during the production process. However, after being prepared into a reagent kit in the present application, it can be directly used in automated equipment to directly prepare the liquid composition of Compound I. The process is simple, the operation is convenient, and the quality of the final product is controllable. Additionally, under the conditions of GMP, standardized management in accordance with the proposed production process requirements, it is possible to consistently and stably produce reagent kits that meet the expected quality standards. This simplifies the entire process and significantly reduces production costs.

### DETAILED DESCRIPTION

Compound I, with the chemical name of 2-tert-butyl-4-chloro-5-((3-((4-((2-(2-fluoro[¹⁸F]ethoxy)ethoxy)methyl)-1*H*-1,2,3-tria zol-1-yl)methyl)benzyl)oxy)pyridazin-3(2*H*)-one, has a chemical structure formula of :

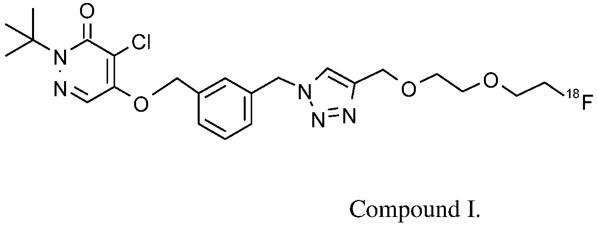

Molecular formula: C₂₃H₂₉Cl¹⁸FN₅O₄; molecular weight: 492.97.

The mechanism of action of Compound I as a myocardial perfusion PET imaging agent: once Compound I enters the myocardial cells, it can quickly interact with the respiratory chain complex I (MC-I) in the mitochondria and remain within the myocardium for an extended period. The data from previous animal studies indicates high cardiac uptake and low liver uptake 15 minutes post-injection, with a favorable heart-to-liver ratio maintained after 60 minutes, demonstrating excellent potential for myocardial perfusion imaging.

In the present application, the liquid composition of Compound I or Compound I is used as a myocardial perfusion PET imaging agent.

Compound I precursor, with the chemical name of methyl 2-(2-((1-(3-(((1-(tert-butyl)-5-chloro-6-oxo-1,6-dihydropyridazin-4-yl)oxy)methyl)be nzyl)-1*H*-1,2,3-triazol-4-yl)methoxy)ethoxy)ethyl-4-toluenesulphonate, has the chemical structure formula of:

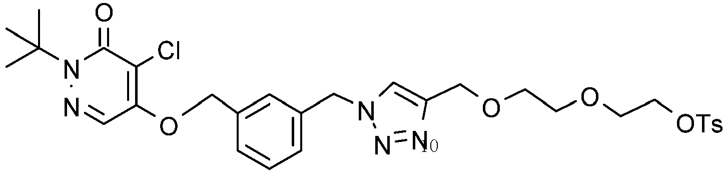

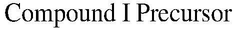

Molecular formula: C₃₀H₃₆ClN₅O₇S; molecular weight: 646.16.

Amino polyether (K₂₂₂) is a triple-bridged crown ether molecule with a hole-like cavity. It is a typical nitrogen-containing cryptand, is one kind of cryptand. Due to its unique coordination properties, nitrogen-containing cryptand can effectively select and complex with cationic transition metals and heavy metals, resulting in more stable complexes, and also possesses both lipophilicity and hydrophilicity, making them promising candidates for further research.

In the prior art, the classic synthesis method of amino polyether (K₂₂₂) is the highly dilute method proposed by Lehn et al., which is one of the typical non-template ion synthesis methods. The specific steps are: dissolving the raw materials 1,8-diamino-3,6-dioxaoctane and 1,8-diacyl chloride-3,6-dioxaoctane in a large amount of benzene solvent, heating and reacting for 8 hours, and then reducing with lithium aluminum tetrahydride for 24 hours, and then separating by column chromatography and recrystallizing to obtain amino polyether (K₂₂₂). This method requires a large amount of solvent, such as benzene, has a long synthetic route, complicated operations, low yield and low economic benefits. In addition to the high dilution method, another classic synthesis method of amino polyether (K₂₂₂) is the synthesis method proposed by Kulstad and Malmsten, which uses Na₂CO₃ and the like as templates to obtain the sodium iodide complex of amino polyether (K₂₂₂) in acetonitrile, and then decomplexed by resin to obtain amino polyether (K₂₂₂). The specific steps are as follows: the raw materials 1,2-di(2-iodoethoxy)ethane and benzylamine are refluxed in acetonitrile solution for 3 days, followed by post-treatment to obtain an intermediate, which is recrystallized with acetone and filtered to obtain a NaI complex. The complex is decomplexed by cation exchange resin and anion exchange resin under acidic condition to prepare an amino polyether (K₂₂₂). The method is simple in equipment, use less solvent and milder in reaction conditions. However, the applicant has found through research that the decomplexation method using ion exchange resins halts when the sodium ion content decreases to a certain level, resulting in low yield.

In this application, the applicant has found that in existing preparation method of Compound I, it is impossible to obtain large scale of liquid composition of Compound I meeting quality requirements, nor products with high activity concentration.

In this application, large scale refer to products with high total activity, generally referring to products with a total activity exceeding 1 Ci, i.e., 37 GBq; high activity concentration products generally refer to products with an activity concentration exceeding 50mCi/mL, i.e., 1850MBq/mL.

In this application, labeling rate refers to the labeling reaction between ¹⁸F and the reaction precursor, where ¹⁸F replaces the leaving group of the precursor and converts it into the final labeled product. The labeled product comprises ¹⁸F, so the labeling rate is defined as the ratio of the activity of the labeled product to the total activity of ¹⁸F participating in the reaction.

In the present application, yield refers to the ratio of activity of the final product liquid composition of Compound I to activity of the initial ¹⁸F.

For example, in the prior art, high-performance liquid chromatography (HPLC) is used for purification and a mixture system of acetonitrile and water is used as the mobile phase in the purification step. The applicant found that if the activity of ¹⁸F is increased, the amount of crude Compound I obtained will increase. If the existing process is still used, the difficulty in purification increases, resulting in a lower labeling rate at high initial ¹⁸F activity; and the yield calculated after purification also decreases, making it impossible to obtain large quantities of Compound I liquid composition that meet the quality requirements. It can be seen from this that using the reaction and purification conditions of the existing technology, simply increasing the starting activity of ¹⁸F ions cannot produce the required high-quality large-scale products. It is evident that when using a mixture system of acetonitrile and water as the mobile phase for the purification of the crude product of Compound I, both the labeling rate and yield are very low.

In addition, the applicant also found that when using a mixture system of acetonitrile and water as the mobile phase to purify the crude product of Compound I, it is necessary to use a C18 column to remove organic solvents such as acetonitrile from the HPLC mobile phase. However, due to the high sensitivity of Compound I to radiolysis, it tends to decompose during purification. During C18 column purification, the radioactive material is concentrated in a very small volume, causing radiolysis. After radiolysis, the yield subsequently decreases. Moreover, the radiochemical purity and stability of the final Compound I product do not meet the requirements, and the preparation time is long. C18 columns are octadecyl-bonded silica gel column that can be used to enrich and remove organic solvents such as acetonitrile.

In order to obtain large scale of liquid composition of Compound I that meet quality requirements, improve the labeling rate and the radiochemical purity and stability of the final product, and shorten the entire reaction time, the applicant has conducted multiple studies and repeated verifications and created a technical solution that can solve the problems of low labeling rate, low radiochemical purity and low stability.

The present application provides a method for preparing Compound I, and the synthetic route is as follows:

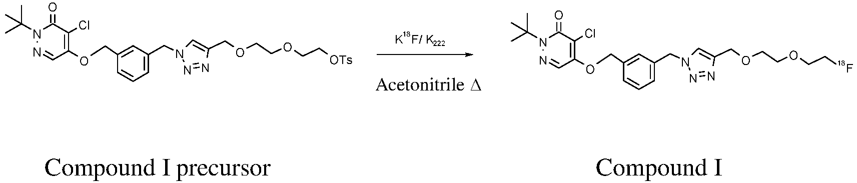

The activated ¹⁸F ion is mixed with an acetonitrile solution comprising a Compound I precursor, and a nucleophilic substitution reaction is carried out in the presence of a catalyst to generate a crude product comprising Compound I.

The aforementioned preparation method of Compound I provided in the present application comprises purifying the crude product comprising Compound I by means of high-performance liquid chromatography (HPLC), wherein the mobile phase used in the HPLC purification step comprises ethanol and water, replacing the original acetonitrile and water system. When using an acetonitrile and water system, a C18 column is required to remove the organic solvent, which results in radiolysis at high activity and cannot meet production needs.

In the above-mentioned preparation method of Compound I provided in the present application, anti-radiolysis agents sodium vitamin C (VcNa) and/or vitamin C (Vc) and/or gentisic acid are added to the mobile phase used in the purification step by high-performance liquid chromatography. Sodium vitamin C, vitamin C and gentisic acid have anti-radiolysis effects. In the HPLC purification process, in order to avoid radiolysis due to the enrichment of radioactive ¹⁸F ions, Vc and/or VcNa and/or gentisic acid are added to the mobile phase to reduce the radiolysis of Compound I during the purification.

The preparation method of Compound I provided in the present application improves the radiochemical purity of the liquid composition of Compound I, eliminates the purification step with C18 column, and reduces the radiolysis in the C18 column.

In the preparation method of the present application, the removal of the C18 column eliminates the need to remove the solvent and allows injectable ethanol to be used as the mobile phase; in addition, using ethanol as the mobile phase can reduce the risk of residual high-risk solvents such as acetonitrile in the liquid composition.

In some specific embodiments of the present application, in the purification step by high-performance liquid chromatography, the ethanol in the mobile phase is 0.2-2 volume parts relative to 1 volume part of water; preferably, the ethanol is 0.4-1 volume part relative to 1 volume part of water in the mobile phase.

For example, relative to 1 volume part of water, the ethanol can be 0.2 volume parts, 0.3 volume parts, 0.4 volume parts, 0.5 volume parts, 0.6 volume parts, 0.7 volume parts, 0.8 volume parts, 0.9 volume parts, 1 volume part, 1.1 volume parts, 1.2 volume parts, 1.3 volume parts, 1.4 volume parts, 1.5 volume parts, 1.6 volume parts, 1.7 volume parts, 1.8 volume parts, 1.9 volume parts, 2 volume parts, or any range therebetween.

In some specific embodiments of the present application, in the purification step by high-performance liquid chromatography, the amount of sodium vitamin C added to the mobile phase is 0-20 mg/mL; preferably, the amount of sodium vitamin C added is 0.2-10 mg/mL;
wherein the amount of sodium vitamin C added can be 0, 2, 5, 8, 10, 12, 15, 20 mg/mL or any range therebetween.

In some specific embodiments of the present application, in the purification step by high-performance liquid chromatography, the amount of vitamin C added to the mobile phase is 0-10 mg/mL; preferably, the amount of vitamin C added is 0.1-5 mg/mL;
the amount of vitamin C added can be 0, 0.1, 0.5, 1, 2, 5, 8, 9, 10 mg/mL or any range therebetween.

In some specific embodiments of the present application, in the purification step by high-performance liquid chromatography, the amount of gentisic acid added to the mobile phase is 0-10 mg/mL; preferably, the amount of gentisic acid added is 0.1-5 mg/mL;
the amount of gentisic acid added can be 0, 0.1, 0.5, 1, 2, 5, 8, 9, 10 mg/mL or any range therebetween.

In some embodiments of the present application, the chromatographic column is a silica gel column, preferably a reverse phase C18 silica gel chromatographic column, and more preferably an XBridge BEH C18 OBD Prep column. This chromatographic column is selected because it is resistant to acid and alkali and has good separation.

In some embodiments of the present application, in the purification step, isocratic elution is employed, with the elution flow rate of the mobile phase being 3-6 mL/min, for example, the elution flow rate of the mobile phase can be 3, 4, 5, 6 mL/min, or any range therebetween.

In some embodiments of the present application, before the purification step by high-performance liquid chromatography, there is also a step of nucleophilic substitution reaction; wherein, in the nucleophilic substitution reaction: the activated ¹⁸F ions are mixed with a solution comprising a Compound I precursor for the nucleophilic substitution reaction to generate a crude product comprising Compound I.

The nucleophilic substitution reaction between the activated ¹⁸F ions and the Compound I precursor can be carried out by a method known to those skilled in the art.

In some embodiments of the present application, in the nucleophilic substitution reaction, the reaction solvent is a non-protonic polar solvent, the range of the amount of Compound I precursor / the activity of ¹⁸F ion activity is (0.5-8): 1; the reaction temperature is 90-140°C, the reaction time is 5-60min, and the reaction is in a sealed space;
the unit of the amount of Compound I precursor is mg, and the unit of the activity of ¹⁸F ion is Ci. Wherein, the ratio of the amount of Compound I precursor to the activity of ¹⁸F ion can be 0.5:1, 1:1, 2:1, 3:1, 4:1, 5:1, 6:1, 7:1, 8:1 or any range therebetween; the unit of the amount of Compound I precursor is mg, and the unit of the activity of ¹⁸F ion is Ci;
the reaction temperature may be 90, 100, 110, 120, 130, 140°C or any range therebetween;
the reaction time can be 5, 15, 20, 35, 45, 60 min or any range therebetween.

In some embodiments of the present application, the amount of the reaction solvent is 0.2-5 mL, and the amount of Compound I precursor is 0.8-20 mg, preferably 7-20 mg; wherein, the amount of the Compound I precursor can be 0.8, 2, 6, 7, 8, 12, 18, 20 mg or any range therebetween;

In some embodiments of the present application, the non-protonic polar solvent is one or more selected from the group consisting of acetonitrile, dimethyl sulfoxide, N,N-dimethylformamide, and 2-methyl-2-butanol.

In some embodiments of the present application, the method further comprises a step for preparing ¹⁸F ion before the step of nucleophilic substitution reaction; and the step for preparing ¹⁸F ion further comprises a step for preparing a ¹⁸F ion solution, a step for enriching and eluting ¹⁸F ion, and a step for activating ¹⁸F ion;
wherein, in the step for preparing a ¹⁸F ion solution, the ¹⁸F ion solution is prepared by accelerator;
in the step for enriching ¹⁸F ion, the ¹⁸F ion solution prepared above is enriched by an anion exchange cartridge;
in the step for eluting ¹⁸F ion, ¹⁸F ions are eluted by eluting with a catalyst solution of cryptand and alkali metal salt;
in the step for activating ¹⁸F ion, the solvent is blown dry by program-controlled temperature, nitrogen or other inert gas, and the ¹⁸F ions are activated to obtain the activated ¹⁸F ions.

In some embodiments of the present application, in the step for preparing ¹⁸F ion solution, the water comprising ¹⁸O is transferred to the accelerator target site, and the accelerator is initiated to generate a proton beam to bombard the water comprising ¹⁸O to produce a solution comprising ¹⁸F ions.

In some embodiments of the present application, in the step for enriching ¹⁸F ion, the anion exchange column is Sep-Pak Accell Plus QMA Carbonate Plus Light Cartridge, specifically a tetraalkylammonium salt anion exchange column.

In the present application, the initial activity of ¹⁸F, also known as the activity of ¹⁸F ion, refers to the activity of ¹⁸F ion measured by an activity meter after starting an accelerator to generate a proton beam to bombard oxygen-containing [¹⁸O] water to produce a solution comprising ¹⁸F ions.

In the present application, the initial activity of ¹⁸F refers to the concentration that is detectable after producing a solution comprising ¹⁸F ions by using a starting accelerator to generate a proton beam to bombard water containing oxygen [¹⁸O]. That is, detectable refers to a reasonable detection time that can be controlled by people skilled in the art, for example, within 10 minutes after production. In addition, those skilled in the art will understand that the initial activity of ¹⁸F will vary with the change of post-production storage time, but an error range of ±10% is acceptable.

In some embodiments of the present application, the activity of ¹⁸F ion is 0.045Ci-11Ci; preferably 3.15Ci-11Ci; for example, the activity of ¹⁸F ion can be 0.045Ci, 0.05Ci, 1Ci, 3Ci, 3.15Ci, 5Ci, 6Ci, 8Ci, 9Ci, 10Ci, 11Ci or any range therebetween.

In some embodiments of the present application, in the step for eluting ¹⁸F ion, in the catalyst solution of cryptand and alkali metal salt, the amount of cryptand is 5-40 mg, and the amount of alkali metal salt is 1.5-20 mg; the amount of cryptand in the solution can be 5, 8, 10, 15, 20, 40 mg or any range therebetween; the amount of alkali metal salt in the solution can be 1.5, 3, 5, 10, 20 mg or any range therebetween.

In some embodiments of the present application, the cryptand is 4,7,13,16,21,24-hexaoxa-1,10-diazabicyclo[8,8,8]hexacosane (Kryptofix-2.2.2, amino polyether); the alkali metal salt is one or more selected from the group consisting of K₂CO₃, Na₂CO₃, Cs₂CO₃, KHCO₃, and NaHCO₃.

In some embodiments of the present application, the catalyst solution is selected from a mixed solvent system of acetonitrile and water, wherein the volume ratio of acetonitrile to water is (0.2-10):1; for example, the volume ratio of acetonitrile to water can be 0.2:1, 1:1, 2:1, 4:1, 7:1, 10:1 or any range therebetween; the volume of the mixed solvent of acetonitrile and water is 0.3-2 mL.

In some embodiments of the present application, in the step for activating ¹⁸F ion, the activation temperature is 80-130°C;
wherein temperature controlling by program comprises the following steps: evaporating for 60-120s under the condition of 100-120°C, positive pressure of 50-200mbar, and vacuum pressure -20~-60mbar; evaporating for 150-200s 1 under the condition of 20-130°C, positive pressure of 50-200mbar, and vacuum pressure -20~-60mbar; evaporating for 10-30s under the condition of 120-130°C, positive pressure of 50-200mbar, and vacuum pressure of -60~-100mbar,; evaporating for 80-120s under the condition of 100-120°C, positive pressure of 800-1200mbar, and vacuum pressure of -800~-1000mbar; evaporating for 100-120s under the condition of 80-100°C, positive pressure of 400-600mbar, and vacuum pressure of -800~ -1000mbar; and evaporating for 10-20s under the condition of 80-100°C, positive pressure of 600-900mbar, and vacuum pressure of -800~-1000mbar.

The above-mentioned preparation method of Compound I provided in the present application has improved the labeling rate by changing the amount of Compound I precursor and achieving the same labeling rate with a shorter reaction time, thereby improving the yield and obtaining the Compound I product. The process parameters and process flow are clear and specific, suitable for large-scale activity production and meet the automation needs. In addition, the purification process employs anti-radiolysis agent in the mobile phase to avoid product loss due to radiolysis during purification and formulation, resulting in higher yields. In addition, the present application uses an anti-radiolysis agent during the formulation process to ensure the stability of the product.

The increase in reaction solvent is based on the fact that Compound I, required after the reaction is completed, is a lipophilic substance. After diluting with water, a higher proportion of organic phase will reduce the residual Compound I in the reaction flask, thereby improving the yield.

In some embodiments of the present application, the preparation of Compound I comprises the following steps:
1) preparing ¹⁸F ion solution
   the water comprising ¹⁸O is transferred to the accelerator target site, and the accelerator is initiated to generate a proton beam to bombard the water comprising ¹⁸O to produce a solution comprising ¹⁸F ions.
2) enriching ¹⁸F ion
   After the ¹⁸F ion solution prepared above is passed through an anion exchange solid phase extraction column (QMA cartridge of the Waters brand, the QMA cartridge is preferably activated by 1 mol/L NaHCO₃), the ¹⁸F ions are enriched on the QMA cartridge.
3) eluting ¹⁸F ion
   The ¹⁸F ions are eluted into the reaction flask by eluting with catalyst solution of cryptand and alkali metal salt. Specifically, 5-40 mg of K₂₂₂ and 1.5-20 mg of K₂CO₃ are mixed to prepare a mixed solvent system solution of acetonitrile and water (the volume ratio of acetonitrile to water is (0.2-10):1, and the volume of the mixed solvent of acetonitrile and water is 0.3-2 mL). The QMA cartridge is eluted and the K¹⁸F/K₂₂₂ complex is eluted into the reaction flask.
4) activating ¹⁸F ion
   Activated ¹⁸F ions are obtained by heating the eluted ¹⁸F ions from step 3) at 80-130° C under a nitrogen or inert gas flow to evaporate the solvent.
5) Nucleophilic substitution reaction of ¹⁸F ions
   Add 0.2-5 mL of acetonitrile solution of Compound I precursor (the amount of Compound I precursor is 7-20 mg) to the reaction flask, heat to 90-140°C under sealed conditions and react for 5-60 minutes. The Compound I precursor undergoes nucleophilic substitution reaction with K¹⁸F/K₂₂₂ to generate a crude product containing Compound I.
6) Purification by high-performance liquid chromatography
   The crude product containing Compound I is loaded into the sample loop, a certain amount of water is drawn to rinse the reaction flask and then loaded into the sample loop, and purified according to the following chromatographic conditions:
   chromatographic column: reverse phase C18 silica gel column;
   mobile phase: a mixture system of ethanol and water, wherein the volume ratio of ethanol to water is (0.2-2):1, and the mobile phase comprises 0-20 mg/mL of sodium vitamin C and/or 0-10 mg/mL of vitamin C and/or 0-10 mg/mL of gentisic acid;
   flow rate: 3-6mL/min;
   detector: radioactivity detector.

Monitor and track the radioactive signal, and collect the main radioactive peak of Compound I into a transfer bottle; after purification, obtaining the pure Compound I.

In some embodiments of the present application, the mobile phase may comprise 0-20 mg/mL of sodium vitamin C and/or 0-10 mg/mL of vitamin C.

In some embodiments of the present application, the mobile phase may comprise 0-20 mg/mL of sodium vitamin C and/or 0-10 mg/mL of gentisic acid.

In some embodiments of the present application, the mobile phase may comprise both vitamin C and gentisic acid, or may comprise only one of vitamin C and gentisic acid.

The present application further provides the aforementioned liquid composition of Compound I. Compound I is collected into a transfer bottle to which the formulation has been added in advance, and the compound I is mixed with the mobile phase of the main peak of HPLC to obtain a compound I liquid composition; the liquid composition of Compound I comprises one or more of vitamin C, sodium vitamin C and gentisic acid.

In some embodiments of the present application, the ratio of concentration (mg/mL) of vitamin C to activity concentration (mCi/mL) of Compound I is 0.02-0.2; preferably, the ratio of concentration (mg/mL) of vitamin C to activity concentration (mCi/mL) of Compound I is 0.04-0.15; for example, the ratio of concentration (mg/mL) of vitamin C to activity concentration (mCi/mL) of Compound I can be 0.02, 0.04, 0.06, 0.08, 0.1, 0.12, 0.14, 0.16, 0.18, 0.2, or any range therebetween.

In some embodiments of the present application, the ratio of concentration (mg/mL) of gentisic acid to activity concentration (mCi/mL) of Compound I is 0.02-0.2; preferably, the ratio of concentration (mg/mL) of gentisic acid to activity concentration (mCi/mL) of Compound I is 0.04-0.15; for example, the ratio of concentration (mg/mL) of gentisic acid to activity concentration (mCi/mL) of Compound I can be 0.02, 0.04, 0.06, 0.08, 0.1, 0.12, 0.14, 0.16, 0.18, 0.2, or any range therebetween.

In some embodiments of the present application, the ratio of concentration (mg/mL) of sodium vitamin C to activity concentration (mCi/mL) of Compound I is 0.04-1; preferably, the ratio of concentration (mg/mL) of sodium vitamin C to activity concentration (mCi/mL) of Compound I is 0.1-1; for example, the ratio of concentration (mg/mL) of sodium vitamin C to activity concentration (mCi/mL) of Compound I is 0.04, 0.1, 0.2, 0.3, 0.4, 0.5, 0.6, 0.7, 0.8, 0.9, 1 or any range therebetween.

In some embodiments of the present application, the amount of sodium vitamin C is 2-10 parts by weight relative to 1 part by weight of vitamin C; preferably, the amount of sodium vitamin C is 3-10 parts by weight relative to 1 part by weight of vitamin C; for example, relative to 1 part by weight of vitamin C, the amount of sodium vitamin C can be 2 parts by weight, 3 parts by weight, 4 parts by weight, 5 parts by weight, 6 parts by weight, 7 parts by weight, 8 parts by weight, 9 parts by weight, 10 parts by weight or any range therebetween.

In some embodiments of the present application, the amount of gentisic acid is 0.1-5 parts by weight relative to 1 part by weight of vitamin C; preferably, the amount of gentisic acid is 0.1-3 parts by weight relative to 1 part by weight of vitamin C; for example, relative to 1 part by weight of vitamin C, the amount of gentisic acid can be 0.1 parts by weight, 0.5 parts by weight, 1 part by weight, 1.5 parts by weight, 2 parts by weight, 2.5 parts by weight, 3 parts by weight, 3.5 parts by weight, 4 parts by weight, 4.5 parts by weight, 5 parts by weight or any range therebetween.

In some embodiments of the present application, the liquid composition of Compound I may comprise both vitamin C and gentisic acid, or may comprise only one of vitamin C and gentisic acid.

In some embodiments of the present application, the liquid composition of Compound I further comprises an aqueous solution of polyethylene glycol and/or ethanol, and the concentration of the polyethylene glycol is 0.1-0.3 g/mL; preferably, the concentration of the polyethylene glycol is 0.1-0.2 g/mL; for example, the concentration of the polyethylene glycol is 0.1, 0.2, 0.3 g/mL or any range therebetween.

In some embodiments of the present application, the ethanol concentration in the aqueous solution of ethanol is 0.05-0.20 mL/mL; preferably, the ethanol concentration in the aqueous solution of ethanol is 0.05-0.15 mL/mL; the ethanol concentration in the aqueous solution of ethanol is 0.05, 0.10, 0.15, 0.20 mL/mL or any range therebetween.

In some embodiments of the present application, the polyethylene glycol is polyethylene glycol 400.

The present application also provides a method for preparing a liquid composition of Compound I, wherein polyethylene glycol substances and/or ethanol, and one or more of vitamin C, sodium vitamin C, gentisic acid are dissolved in water and stirred to obtain a formulation of a liquid composition of Compound I.

In some embodiments of the present application, the purified Compound I is collected into the prescription bottle to which the aforementioned liquid composition has been added in advance; this is completed when the liquid composition is mixed with the main peak mobile phase in HPLC purification.

The present application also provides the use of the above-mentioned liquid composition of Compound I as a myocardial perfusion PET imaging agent.

In addition, the present application provides a reagent kit for automated preparation of liquid composition of Compound I, wherein the reagent kit comprises a reaction flask and a prescription bottle; the reaction flask includes reaction flask R1, reaction flask R2, and reaction flask R3; the reaction flask R1 is used to hold the eluent for eluting ¹⁸F ion; the reaction flask R2 is used to hold a solution of Compound I precursor for the ¹⁸F ion nucleophilic substitution reaction; the reaction flask R3 is used to hold reagents for diluting the crude products and rinsing the reaction system; the prescription bottle includes prescription bottle P1 and prescription bottle P2; the prescription bottle P1 is used to transfer the reaction products of ¹⁸F ion nucleophilic substitution reaction; the prescription bottle P2 is used to hold substances used to stabilize the reaction products of ¹⁸F ion nucleophilic substitution reaction.

In some embodiments of the present application, the reaction flask R1 comprises amino polyether, potassium carbonate, water for injection, and acetonitrile; preferably, the concentration of the amino polyether is 5-40mg/mL, the concentration of potassium carbonate is 1.5-20mg/mL, and the volume ratio of acetonitrile to water is (0.25-19): 1; for example, the concentration of the amino polyether can be 5mg/mL, 6mg/mL, 7mg/mL, 8mg/mL, 9mg/mL, 10mg/mL, 11mg/mL, 12mg/mL, 13mg/mL, 14mg/mL, 15mg/mL, 16mg/mL, 17mg/mL, 18mg/mL, 19mg/mL, 20mg/mL, 21mg/mL, 22mg/mL, 23mg/mL, 24mg/mL, 25mg/mL, 26mg/mL, 27mg/mL, 28mg/mL, 29mg/mL, 30mg/mL, 31mg/mL, 32mg/mL, 33mg/mL, 34mg/mL, 35mg/mL, 36mg/mL, 37mg/mL, 38mg/mL, 39mg/mL, 40mg/mL or any range therebetween; the concentration of potassium carbonate can be 1.5mg/mL, 2mg/mL, 3mg/mL, 4mg/mL, 5mg/mL, 6mg/mL, 7mg/mL, 8mg/mL, 9mg/mL, 10mg/mL, 11mg/mL, 12mg/mL, 13mg/mL, 14mg/mL, 15mg/mL, 16mg/mL, 17mg/mL, 18mg/mL, 19mg/mL, 20mg/mL or any range therebetween; the volume ratio of acetonitrile to water can be 0.25:1, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1 or any range therebetween.

In some embodiments of the present application, the reaction flask R2 comprises an acetonitrile solution of the Compound I precursor for ¹⁸F ion nucleophilic substitution reaction; preferably, the concentration of Compound I precursor in the acetonitrile solution is 1-10 mg/mL; for example, the concentration of Compound I precursor in the acetonitrile solution can be 1mg/mL, 2mg/mL, 3mg/mL, 4mg/mL, 5mg/mL, 6mg/mL, 7mg/mL, 8mg/mL, 9mg/mL, 10mg/mL, or any range therebetween.

In some embodiments of the present application, the reaction flask R3 comprises anhydrous ethanol.

In some embodiments of the present application, the prescription bottle P1 comprises polyethylene glycol; preferably, the concentration of polyethylene glycol is 0.05-0.3 g/mL; for example, the concentration of polyethylene glycol can be 0.05g/mL, 0.1g/mL, 0.15g/mL, 0.2g/mL, 0.25g/mL, 0.3g/mL, or any range therebetween.

In some embodiments of the present application, the prescription bottle P2 comprises one or more of vitamin C, sodium vitamin C, and gentisic acid for stabilizing the reaction products of the ¹⁸F ion nucleophilic substitution reaction; wherein vitamin C, sodium vitamin C and gentisic acid are dissolved in water before use; preferably, the concentration of vitamin C is 2-16mg/mL; the concentration of sodium vitamin C is 15-40mg/mL, and the concentration of gentisic acid is 2-16mg/mL; for example, the concentration of vitamin C can be 2mg/mL, 3mg/mL, 4mg/mL, 5mg/mL, 6mg/mL, 7mg/mL, 8mg/mL, 9mg/mL, 10mg/mL, 11mg/mL, 12mg/mL, 13mg/mL, 14mg/mL, 15mg/mL, 16mg/mL or any range therebetween; the sodium vitamin C can be 15mg/mL, 16mg/mL, 17mg/mL, 18mg/mL, 19mg/mL, 20mg/mL, 21mg/mL, 22mg/mL, 23mg/mL, 24mg/mL, 25mg/mL, 26mg/mL, 27mg/mL, 28mg/mL, 29mg/mL, 30mg/mL, 31mg/mL, 32mg/mL, 33mg/mL, 34mg/mL, 35mg/mL, 36mg/mL, 37mg/mL, 38mg/mL, 39mg/mL, 40mg/mL or any range therebetween; the concentration of gentisic acid can be 2mg/mL, 3mg/mL, 4mg/mL, 5mg/mL, 6mg/mL, 7mg/mL, 8mg/mL, 9mg/mL, 10mg/mL, 11mg/mL, 12mg/mL, 13mg/mL, 14mg/mL, 15mg/mL, 16mg/mL or any range therebetween.

Reaction flasks R1, R2, R3, prescription bottles P1, and P2 can be formulated according to actual needs, as long as their concentration ranges meeting the requirements.

The present application provides a general and/or specific description of the materials and experimental methods used in the experiments. In the following examples, unless otherwise specified, % means wt%, i.e., weight percentage. The reagents or instruments used without indicating the manufacturer are all conventional reagent products that can be commercially obtained. Table 1 shows the sources of the raw materials used in the examples.

**Table 1: Sources of the raw materials used in the examples**

| raw materials | Purity/Model | Manufacturer |
|---|---|---|
| Compound I precursor | > 95% | Homemade |
| Sodium Vitamin C | ≥99.0% | CSPC Pharmaceutical Group Weisheng Pharmaceutical (Shijiazhuang) Co., Ltd |
| Vitamin C | ≥99.0% | CSPC Pharmaceutical Group Weisheng Pharmaceutical (Shijiazhuang) Co., Ltd. |
| gentisic acid | ≥98.0% | Homemade |
| Anhydrous ethanol | Pharmaceutical excipients | Hunan Xiangyikang Pharmaceutical Co., Ltd. |
| C18 OBD Chromatographic column | XBridge BEH C18 OBD Prep column,130A,5µm,10×250mm | Waters |
| cryptand K₂₂₂ | GMP level | ABX |
| NaHCO₃ | ≥99.0% | Energy Chemical (Beijing) |
| K₂CO₃ | ≥99.0% | Sigma-Aldrich |
| PEG400 | Pharmaceutical excipients | Nanjing Weir Biotechnology Co., Ltd. |
| Sterile Injection Water | Injection grade | SJZ Siyao Co., Ltd. |

### Example

The automation equipment used is the AllinOne equipment from Trasis. The power unit of the equipment consists of high-purity nitrogen and an electric rotor for injectors, which can provide a vacuum system and is equipped with an HPLC purification system. Due to the use of automated equipment in this process, which is housed within a radiation shielding box, operators are protected from radiation exposure, allowing for increased operational amounts. Additionally, computer control ensures more precise and repeatable process steps, reducing human deviation.

### Example 1: Preparation of Compound I

### 1) Preparation of ¹⁸F ion solution

2 g of water comprising ¹⁸O was transferred to the accelerator target site, and the accelerator was initiated to generate a proton beam to bombard the water comprising ¹⁸O to produce a solution comprising ¹⁸F ions. In this example, a large amount of initial ¹⁸F was used, and the initial activity of ¹⁸F was 3.5Ci.

### 2) ¹⁸F ion enrichment

After the ¹⁸F ion solution prepared above was passed through an anion exchange solid phase extraction column (QMA column of the Waters brand, the QMA column was preferably activated by 1 mol/L NaHCO₃), the ¹⁸F ions were enriched on the QMA column.

### 3) ¹⁸F ion elution

The ¹⁸F ions were eluted into the reaction flask by eluting with catalyst solution of cryptand and alkali metal salt. Specifically, 15 mg of K₂₂₂ (dissolved in 0.9mL acetonitrile) and 1.5 mg of K₂CO₃ (dissolved in 0.1mL water) were mixed to prepare a mixed solvent solution of acetonitrile and water (the volume ratio of acetonitrile to water is 9:1 (i.e., reaction flask R1, the formulation and preparation of which are described in the preparation of the reaction flask R1); the QMA column was eluted and the K¹⁸F/K₂₂₂ complex was eluted into the reaction flask.

### 4) ¹⁸F ion activation

Activated ¹⁸F ions were obtained by heating the eluted ¹⁸F ions from step 3) at 80-130° C under a nitrogen or inert gas flow to evaporate the solvent.

### 5) Nucleophilic substitution reaction of ¹⁸F ions

Added 3 mL of acetonitrile solution of Compound I precursor (the amount of Compound I precursor is 12 mg) to the reaction flask (i.e., reaction flask R2, the formulation and preparation of which are described in the preparation of the reaction flask R2), heated to 100°C under sealed conditions and reacted for 10 minutes. The Compound I precursor underwent nucleophilic substitution reaction with K¹⁸F/K₂₂₂ to generate a crude product containing Compound I.

### 6) Purification by high-performance liquid chromatography

The crude product containing Compound I was loaded into the sample loop, 2.5mL water was drawn to rinse the reaction flask and then loaded into the sample loop, and purified according to the following chromatographic conditions:
chromatographic column: XBridge BEH C18 OBD Prep column, 130A, 5µm, 10×250mm;
mobile phase: a mixture system of ethanol and water, wherein the volume ratio of ethanol to water was 0.6:1, and the mobile phase further comprised 5mg/mL of sodium vitamin C and/or 1mg/mL of vitamin C (the mobile phase can be obtained from the prescription bottle P2, the formulation and preparation of which are described in the preparation of the prescription bottle P2);
flow rate: 4mL/min;
detector: radioactivity detector.

Monitored and tracked the radioactive signal, and collected the main radioactive peak of Compound I into a transfer bottle; after purification, obtained the pure Compound I.

### Example 2

The only difference between Example 2 and Example 1 is: 5) in the ¹⁸F ion nucleophilic substitution reaction, the amount of Compound I precursor was 20mg.

### Example 3

The only difference between Example 3 and Example 2 is: 1) in the preparation of the ¹⁸F ion solution, the initial activity of ¹⁸F was 6Ci.

### Example 4

The only difference between Example 4 and Example 2 is: 1) in the preparation of the ¹⁸F ion solution, the initial activity of ¹⁸F is 10Ci.

### Example 5

The only difference between Example 5 and Example 4 is: 6) in the purification by high-performance liquid chromatography, the mobile phase was a mixture system of ethanol and water, wherein the volume ratio of ethanol to water was 2:1, and the mobile phase further comprised sodium vitamin C with a concentration of 5 mg/mL and vitamin C with a concentration of 1 mg/mL.

### Example 6

The only difference between Example 6 and Example 4 is: 6) in the purification by high-performance liquid chromatography, the mobile phase was a mixture system of ethanol and water, wherein the volume ratio of ethanol to water was 1:1, and the mobile phase further comprised sodium vitamin C with a concentration of 5 mg/mL and vitamin C with a concentration of 1 mg/mL.

### Example 7

The only difference between Example 7 and Example 4 is: 6) in the purification by high-performance liquid chromatography, the mobile phase was a mixture system of ethanol and water, wherein the volume ratio of ethanol to water was 0.6:1, and the mobile phase further comprised sodium vitamin C with a concentration of 5 mg/mL.

### Example 8

The only difference between Example 8 and Example 4 is: 6) in the purification by high-performance liquid chromatography, the mobile phase was a mixture system of ethanol and water, wherein the volume ratio of ethanol to water was 0.6:1, and the mobile phase further comprised sodium vitamin C with a concentration of 5 mg/mL and vitamin C with a concentration of 5 mg/mL.

### Example 9

The only difference between Example 9 and Example 4 is: 6) in the purification by high-performance liquid chromatography, the mobile phase was a mixture system of ethanol and water, wherein the volume ratio of ethanol to water was 0.6:1, and the mobile phase further comprised vitamin C with a concentration of 1 mg/mL.

### Example 10

The only difference between Example 10 and Example 4 is: 6) in the purification by high-performance liquid chromatography, the mobile phase was a mixture system of ethanol and water, wherein the volume ratio of ethanol to water was 0.6:1, and the mobile phase further comprised sodium vitamin C with a concentration of 10 mg/mL and vitamin C with a concentration of 1 mg/mL.

### Example 11

The only difference between Example 11 and Example 4 is: 6) in the purification by high-performance liquid chromatography, the mobile phase was a mixture system of ethanol and water, wherein the volume ratio of ethanol to water was 0.6:1.

### Example 12

The only difference between Example 12 and Example 4 is: 6) in the purification by high-performance liquid chromatography, the mobile phase was a mixture system of ethanol and water, wherein the volume ratio of ethanol to water was 0.6:1, and the mobile phase further comprised sodium vitamin C with a concentration of 5 mg/mL and gentisic acid with a concentration of 1 mg/mL.

### Example 13

The only difference between Example 13 and Example 4 is: 6) in the purification by high-performance liquid chromatography, the mobile phase was a mixture system of ethanol and water, wherein the volume ratio of ethanol to water was 0.6:1, and the mobile phase further comprised gentisic acid with a concentration of 1 mg/mL.

### Example 14

The only difference between Example 14 and Example 4 is: 6) in the purification by high-performance liquid chromatography, the mobile phase was a mixture system of ethanol and water, wherein the volume ratio of ethanol to water was 0.6:1, and the mobile phase further comprised sodium vitamin C with a concentration of 5 mg/mL and L-glutathione with a concentration of 1 mg/mL.

### Example 15

The only difference between Example 15 and Example 4 is: 6) in the purification by high-performance liquid chromatography, the mobile phase was a mixture system of ethanol and water, wherein the volume ratio of ethanol to water was 0.6:1, and the mobile phase further comprised thiourea with a concentration of 1 mg/mL and sodium vitamin C with a concentration of 5 mg/mL.

### Example 16

The only difference between Example 16 and Example 4 is: 6) in the purification by high-performance liquid chromatography, the mobile phase was a mixture system of ethanol and water, wherein the volume ratio of ethanol to water was 1:9, and the mobile phase further comprised sodium vitamin C with a concentration of 5 mg/mL and vitamin C with a concentration of 1 mg/mL.

### Comparative Example 1

The only difference between Comparative Example 1 and Example 4 is: 6) in the purification by high-performance liquid chromatography, the mobile phase was a mixture system of acetonitrile and water, wherein the volume ratio of acetonitrile to water was 0.6:1, and the mobile phase further comprised sodium vitamin C with a concentration of 5 mg/mL and vitamin C with a concentration of 1 mg/mL.

### Comparative Example 2

The only difference between Comparative Example 2 and Example 4 is: 6) in the purification by high-performance liquid chromatography, the mobile phase was pure ethanol and did not comprise vitamin C, sodium vitamin C or gentisic acid.

The parameters of Examples 1-16 and Comparative Examples 1-2 are shown in Table 2.

**Table 2: Parameters of Examples 1-16 and Comparative Examples 1-2**

| | ¹⁸F ion solution | ¹⁸F ion nucleophilic substitution reaction | | | Mobile phase of HPLC purification | | | |
|---|---|---|---|---|---|---|---|---|
| | Starting activity of ¹⁸F (Ci) | Precursor amount (mg) | Reaction temperatu re (°C) | Reaction time (min) | Ethanol and water | Vitamin C (Vc)(mg/mL) | Sodium vitamin C (mg/mL) | Flow rate (mL/ min) |
| Example 1 | 3.5 | 12 | 100 | 10 | The volume ratio of ethanol to water is 0.6:1. | 1 | 5 | 4 |
| Example 2 | 3.5 | 20 | 100 | 10 | The volume ratio of ethanol to water is 0.6:1 | 1 | 5 | 4 |
| Example 3 | 6 | 20 | 100 | 10 | The volume ratio of ethanol to water is 0.6:1 | 1 | 5 | 4 |
| Example 4 | 10 | 20 | 100 | 10 | The volume ratio of ethanol to water is 0.6:1 | 1 | 5 | 4 |
| Example 5 | 10 | 20 | 100 | 10 | The volume ratio of ethanol to water is 2:1 | 1 | 5 | 4 |
| Example 6 | 10 | 20 | 100 | 10 | The volume ratio of ethanol to water is 1:1 | 1 | 5 | 4 |
| Example 7 | 10 | 20 | 100 | 10 | The volume ratio of ethanol to water is 0.6:1 | --- | 5 | 4 |
| Example 8 | 10 | 20 | 100 | 10 | The volume ratio of ethanol to water is 0.6:1 | 5 | 5 | 4 |
| Example 9 | 10 | 20 | 100 | 10 | The volume ratio of ethanol to water is 0.6:1 | 1 | --- | 4 |
| Example 10 | 10 | 20 | 100 | 10 | The volume ratio of ethanol to water is 0.6:1 | 1 | 10 | 4 |
| Example 11 | 10 | 20 | 100 | 10 | The volume ratio of ethanol to water is 0.6:1 | --- | --- | 4 |
| Example 12 | 10 | 20 | 100 | 10 | The volume ratio of ethanol to water is 0.6:1 | Vc was replaced by gentisic acid 1mg/mL | 5 | 4 |
| Example 13 | 10 | 20 | 100 | 10 | The volume ratio of ethanol to water is 0.6:1 | Vc was replaced by gentisic acid 1mg/mL | --- | 4 |
| Example 14 | 10 | 20 | 100 | 10 | The volume ratio of ethanol to water is 0.6:1 | Vc was replaced by L-glutathione 1mg/mL | 5 | 4 |
| Example 15 | 10 | 20 | 100 | 10 | The volume ratio of ethanol to water is 0.6:1 | VC was replaced by thiourea 1mg/mL | 5 | 4 |
| Example 16 | 10 | 20 | 100 | 10 | The volume ratio of ethanol to water is 1:9 | 1 | 5 | 4 |
| Comparative Example 1 | 10 | 20 | 100 | 10 | The volume ratio of acetonitrile to water is 0.6:1 | 1 | 5 | 4 |
| Comparative Example 2 | 10 | 20 | 100 | 10 | Ethanol | --- | --- | 4 |

The data on initial activity of ¹⁸F shown in Table 2 refer to data that can be detected immediately after production, but those skilled in the art can understand that the initial activity of ¹⁸F usually varies with the storage time and usage conditions. Therefore, the data on the initial activity of ¹⁸F in Examples 1-16 and Comparative Examples 1-2 typically fell within ±10% of the target initial activity of ¹⁸F, all of which are within the acceptable range for those skilled in the art. For example, 10Ci was the target initial activity of ¹⁸F, and in actual detection, the initial activity could be 9Ci-11Ci; 6Ci was the target initial activity of ¹⁸F, and in actual detection, the initial activity could be 5.4Ci-6.6Ci; and 3.5Ci was the target initial activity of ¹⁸F, in actual detection, the initial activity could be 3.15Ci-3.85Ci.

### Example 17: Liquid Composition of Compound I

Took the Compound I product of Example 1, formulated it into a liquid composition of Compound I, and collected Compound I into a transfer bottle (i.e., prescription bottle P1. The formula and preparation of the prescription bottle P1 refer to the preparation of prescription bottle P1, wherein the formula matrix liquid is as follows: reagents in another prescription bottle P2 was dissolved in water to a specified volume in advance, took a portion of the prepared reagent from the other prescription bottle P2; the reagent taken from the other prescription bottle P2 was mixed with the reagent in prescription bottle P1 to form the formula matrix liquid) into which the formula matrix had been added in advance. Then, the remaining portion of vitamin C and sodium vitamin C from the other prescription bottle P2 (i.e., the prescription bottle P2, the formula and preparation of which refer to the preparation of prescription bottle P2) was added to prescription bottle P1. After mixing, the activity concentration of the pure Compound I was 2000 MBq/mL, i.e. 54 mCi/mL. The liquid composition was mixed with the mobile phase of the main peak of HPLC to complete the formulation. Specifically, the formula comprised 0.1 g/mL of polyethylene glycol 400, 2 mg/mL of vitamin C (wherein the concentration (mg/mL) of vitamin C / the activity concentration (mCi/mL) of Compound I was 0.04), 20 mg/mL of sodium vitamin C (wherein the concentration (mg/mL) of sodium vitamin C / the activity concentration (mCi/mL) of Compound I was 0.37), 0.816 mL/mL of water, and 0.094 mL/mL of ethanol (the mass ratio of the vitamin C to the sodium vitamin C was 1:10).

### Example 18

The difference between Example 18 and Example 17 is: the formula comprised 0.1 g/mL of polyethylene glycol 400, 6 mg/mL of vitamin C (wherein the concentration (mg/mL) of vitamin C / the activity concentration (mCi/mL) of Compound I was 0.11), 15 mg/mL of sodium vitamin C (wherein the concentration (mg/mL) of sodium vitamin C / the activity concentration (mCi/mL) of Compound I was 0.28), 0.816 mL/mL of water, and 0.094 mL/mL of ethanol (the mass ratio of the vitamin C to the sodium vitamin C was 2:5).

### Example 19

The difference between Example 19 and Example 17 is: the formula comprised 0.1 g/mL of polyethylene glycol 400, 2 mg/mL of vitamin C (wherein the concentration (mg/mL) of vitamin C / the activity concentration (mCi/mL) of Compound I was 0.04), 40 mg/mL of sodium vitamin C (wherein the concentration (mg/mL) of sodium vitamin C / the activity concentration (mCi/mL) of Compound I was 0.74), 0.816 mL/mL of water, and 0.094 mL/mL of ethanol (the mass ratio of the vitamin C to the sodium vitamin C was 1:20).

### Example 20

The difference between Example 20 and Example 17 is: the formula comprised 0.1 g/mL of polyethylene glycol 400, 8 mg/mL of vitamin C (wherein the concentration (mg/mL) of vitamin C / the activity concentration (mCi/mL) of Compound I was 0.15), 20 mg/mL of sodium vitamin C (wherein the concentration (mg/mL) of sodium vitamin C / the activity concentration (mCi/mL) of Compound I was 0.37), 0.816 mL/mL of water, and 0.094 mL/mL of ethanol (the mass ratio of the vitamin C to the sodium vitamin C was 2:5).

### Example 21

The difference between Example 21 and Example 17 is: the formula comprised 0.1 g/mL of polyethylene glycol 400, 16 mg/mL of vitamin C (wherein the concentration (mg/mL) of vitamin C / the activity concentration (mCi/mL) of Compound I was 0.3), 40 mg/mL of sodium vitamin C (wherein the concentration (mg/mL) of sodium vitamin C / the activity concentration (mCi/mL) of Compound I was 0.74), 0.816 mL/mL of water, and 0.094 mL/mL of ethanol (the mass ratio of the vitamin C to the sodium vitamin C was 2:5).

### Example 22

The difference between Example 22 and Example 17 is: the formula comprised 0.1 g/mL of polyethylene glycol 400, 2 mg/mL of gentisic acid (wherein the concentration (mg/mL) of gentisic acid / the activity concentration (mCi/mL) of Compound I was 0.04), 20 mg/mL of sodium vitamin C (wherein the concentration (mg/mL) of sodium vitamin C / the activity concentration (mCi/mL) of Compound I was 0.37), 0.816 mL/mL of water, and 0.094 mL/mL of ethanol (the mass ratio of gentisic acid to the sodium vitamin C was 1:10).

### Comparative Example 3

The difference between Comparative Example 3 and Example 17 is: the formula comprised 0.1 g/mL of polyethylene glycol 400, 0.816 mL/mL of water, and 0.094 mL/mL of ethanol.

### Comparative Example 4

The difference between Comparative Example 4 and Example 17 is that the formula comprised 0.1 g/mL of polyethylene glycol 400, 20 mg/mL of sodium vitamin C (wherein the concentration (mg/mL) of sodium vitamin C / the activity concentration (mCi/mL) of Compound I was 0.37), 0.816 mL/mL of water, and 0.094 mL/mL of ethanol.

### Comparative Example 5

The difference between Comparative Example 5 and Example 17 is: the formula comprised 0.1 g/mL of polyethylene glycol 400, 2 mg/mL of vitamin C (wherein the concentration (mg/mL) of vitamin C / the activity concentration (mCi/mL) of Compound I was 0.04), 0.816 mL/mL of water, and 0.094 mL/mL of ethanol.

### Comparative Example 6

The difference between Comparative Example 6 and Example 17 is: the formula comprised 0.1 g/mL of polyethylene glycol 400, 2 mg/mL of L-glutathione (wherein the concentration (mg/mL) of L-glutathione / the activity concentration (mCi/mL) of Compound I was 0.04), 20 mg/mL of sodium vitamin C (wherein the concentration (mg/mL) of sodium vitamin C / the activity concentration (mCi/mL) of Compound I was 0.37), 0.816 mL/mL of water, and 0.094 mL/mL of ethanol (the mass ratio of the L-glutathione to the sodium vitamin C was 1:10).

### Comparative Example 7

The difference between Comparative Example 7 and Example 17 is: the formula comprised 0.1 g/mL of polyethylene glycol 400, 2 mg/mL thiourea (wherein the concentration (mg/mL) of thiourea / the activity concentration (mCi/mL) of Compound I was 0.04), 20 mg/mL of sodium vitamin C (wherein the concentration (mg/mL) of sodium vitamin C / the activity concentration (mCi/mL) of Compound I was 0.37), 0.816 mL/mL of water, and 0.094 mL/mL of ethanol (the mass ratio of the thiourea to the sodium vitamin C was 1:10).

### Comparative Example 8

The difference between Comparative Example 8 and Example 17 is: the formula comprised 0.1 g/mL of polyethylene glycol 400, 2 mg/mL sodium metabisulfite (wherein the concentration (mg/mL) of sodium metabisulfite / the activity concentration (mCi/mL) of Compound I was 0.04), 20 mg/mL of sodium vitamin C (wherein the concentration (mg/mL) of sodium vitamin C / the activity concentration (mCi/mL) of Compound I was 0.37), 0.816 mL/mL of water, and 0.094 mL/mL of ethanol (the mass ratio of the sodium metabisulfite to the sodium vitamin C was 1:10).

The parameters of Examples 17-22 and Comparative Examples 3-8 are shown in Table 3.

**Table 3: Parameters of Examples 17-22 and Comparative Examples 3-8**

| | Activity concentra tion of the product (MBq/m L) | Wate r (mL/ mL) | Ethano l (mL/m L) | polyethyl ene glycol 400 (g/mL) | Vitamin C (Vc) (mg/mL) | Concentrati on of VC (mg/mL)/ Activity Concentrati on of Compound I (mCi/mL) | Sodiu m vitamin C (VcNa) (mg/m L) | Concentrati on of Sodium vitamin C (mg/mL)/ Activity Concentrati on of Compound I (mCi/mL) | Mass ratio of vitamin C to sodium vitamin C |
|---|---|---|---|---|---|---|---|---|---|
| Example 17 | 2000 | 0.81 6 | 0.094 | 0.1 | 2 | 0.04 | 20 | 0.37 | 1:10 |
| Example 18 | 2000 | 0.81 6 | 0.094 | 0.1 | 6 | 0.11 | 15 | 0.28 | 2:5 |
| Example 19 | 2000 | 0.81 6 | 0.094 | 0.1 | 2 | 0.04 | 40 | 0.74 | 1:20 |
| Example 20 | 2000 | 0.81 6 | 0.094 | 0.1 | 8 | 0.15 | 20 | 0.37 | 2:5 |
| Example 21 | 2000 | 0.81 6 | 0.094 | 0.1 | 16 | 0.30 | 40 | 0.74 | 2:5 |
| Example 22 | 2000 | 0.81 6 | 0.094 | 0.1 | Replaced Vc with 2mg/mL gentisic acid. | 0.04 | 20 | 0.37 | 1:10 |
| Comparative example 3 | 2000 | 0.81 6 | 0.094 | 0.1 | --- | --- | --- | --- | --- |
| Comparative example 4 | 2000 | 0.81 6 | 0.094 | 0.1 | --- | --- | 20 | 0.37 | --- |
| Comparative example 5 | 2000 | 0.81 6 | 0.094 | 0.1 | 2 | 0.04 | --- | --- | --- |
| Comparative example 6 | 2000 | 0.81 6 | 0.094 | 0.1 | Replaced Vc with 2mg/mL L-glutathio ne. | 0.04 | 20 | 0.37 | 1:10 |
| Comparative example 7 | 2000 | 0.81 6 | 0.094 | 0.1 | Replaced Vc with 2 mg/mL thiourea. | 0.04 | 20 | 0.37 | 1:10 |
| Comparative example 8 | 2000 | 0.81 6 | 0.094 | 0.1 | Replaced Vc with 2mg/mL sodium metabisulfit e. | 0.04 | 20 | 0.37 | 1:10 |

The activity concentration of the product in Table 3 refers to the activity concentration of pure Compound I, where 1mCi=37MBq. The activity concentration of pure Compound 1 is defined as the value obtained by dividing the activity of the Compound 1 product by the total volume of the liquid composition of Compound 1.

In Example 17, the activity concentration of the product was calculated as 2000 MBq/mL, i.e. 54mCi/mL, the ratio of the concentration (mg/mL) of vitamin C to the activity concentration (mCi/mL) of Compound I was 2 to 54, i.e. 0.04, and the ratio of the concentration (mg/mL) of odium vitamin C to the activity concentration (mCi/mL) of Compound I was 20 to 54, i.e. 0.37.

### Experimental example

The method for determining the labeling rate was: after the labeling reaction was completed, high performance liquid chromatography (HPLC) was used for sample analysis, and the ratio of the peak area of the radioactive target product to the peak area of all radioactive peaks in the liquid chromatogram was obtained.

The yield without decay correction was determined by the ratio of the activity of the final liquid composition product measured by an activity meter to the initial activity of ¹⁸F.

The method for determining radiochemical purity at 0h involved using HPLC for sample analysis, where the ratio of the product's radioactive peak area to the peak area of all radioactive peaks was calculated.

The method for determining the 6h stability (radiochemical purity index) involved placing the final product at room temperature for 6 hours, then analyzing it using HPLC sample analysis, and calculating the ratio of the product's radioactive peak area to the total peak area of all radioactive peaks.

The experimental effect data of Examples 1-16 and Comparative Examples 1-2 are shown in Table 4.

**Table 4: Experimental effect data of Examples 1-16 and Comparative Examples 1-2**

| | Labeling rate (%) | Total preparation time (min) | Yield without decay correction (%) | Radiochemical purity at 0h (%) |
|---|---|---|---|---|
| Example 1 | 73.8 | 48 | 38.0 | 98.2 |
| Example 2 | 79.7 | 49 | 43.8 | 98.9 |
| Example 3 | 78.7 | 48 | 42.7 | 98.3 |
| Example 4 | 78.3 | 48 | 42.1 | 98.9 |
| Example 5 | 79.0 | 41 | 43.0 | 86.8 |
| Example 6 | 78.5 | 44 | 42.6 | 93.4 |
| Example 7 | 72.4 | 48 | 37.0 | 96.0 |
| Example 8 | 79.5 | 49 | 43.5 | 98.1 |
| Example 9 | 71.2 | 48 | 32.1 | 95.6 |
| Example 10 | 79.6 | 49 | 43.7 | 98.2 |
| Example 11 | 65.4 | 49 | 27.9 | 92.1 |
| Example 12 | 77.8 | 48 | 41.5 | 97.8 |
| Example 13 | 71.2 | 48 | 31.1 | 94.9 |
| Example 14 | 72.6 | 48 | 37.2 | 95.6 |
| Example 15 | 72.8 | 50 | 37.5 | 95.8 |
| Example 16 | 58.5 | 70 | 18.6 | 83.4 |
| Comparative example 1 | 62.4 | 62 | 22.5 | 91.4 |
| Comparative example 2 | - | - | - | - |

As can be seen from Table 4, after the HPLC purification process was optimized, the amount of crude product comprising Compound I that can be processed in Examples 1-4 was increased, thereby increasing the reaction amount of Compound I precursor and ¹⁸F initial activity, making it possible to provide a large-scale, high-quality liquid composition of Compound I.

Compared with Example 1, when the initial activity of ¹⁸F was constant, the increase in the amount of compound I precursor in Example 2 promotes the conversion of ¹⁸F, leading to improvements in yield and labeling rate.

In Examples 2-4, the amount of the Compound I precursor was constant, with the increase of the initial activity of ¹⁸F, the yield and labeling rate did not change much, and the radiochemical purity did not change much.

Generally speaking, when the amount of the Compound I precursor is constant, within a certain range, the higher the initial activity of ¹⁸F, the lower the yield; however, once the amount of Compound I precursor reaches a certain level, the yield will no longer change with the variation in the starting labeling amount of ¹⁸F.

In Examples 4-6, the effect of the volume ratio of ethanol to water in the mobile phase on the final efficiency was investigated. When the amount of ethanol in the mobile phase is larger, the final product comes out faster, so the total preparation time will be shorter and the yield will be higher, so the yield of Example 5 and Example 6 was slightly greater than that of Example 4; however, excessive ethanol content can lead to a decrease in the radiochemical purity of the final product, so the radiochemical purity of Example 5 was lower than that of Examples 4 and 6.

In Examples 4, 7, and 8, there was no vitamin C in the mobile phase of Example 7, so the yield and radiochemical purity of Example 7 were lower than those of Example 4 and Example 8; the content of vitamin C in Example 8 was increased compared to that of Example 4, so the yield of Example 8 was higher than that of Example 4.

In Examples 4 and 9-11, compared with Example 4, the mobile phase of Example 9 did not comprise sodium vitamin C, so the yield and radiochemical purity of Example 9 were lower than those of Example 4. The content of sodium vitamin C in Example 10 was higher than that in Example 4, so the yield of Example 10 was better than that of Example 4. There were no sodium vitamin C and vitamin C in the mobile phase of Example 11, resulting in decreased yield, labeling rate and radiochemical purity.

In Examples 12 and 13: compared with Example 4, Example 12 replaced vitamin C with gentisic acid, the labeling rate, yield, and radiochemical purity were comparable to those in Example 4, indicating that gentisic acid functioned similarly to vitamin C in the mobile phase. Compared with Example 12, the mobile phase of Example 13 did not contain sodium vitamin C, so the yield, labeling rate and radiochemical purity of Example 13 were lower than those of Example 12.

Compared with Example 4, vitamin C was replaced with other substances in Examples 14 and 15, the labeling rate, yield and radiochemical purity were all reduced, indicating that vitamin C was more suitable for use in the mobile phase than L-glutathione and thiourea.

In Example 16, the ratio of ethanol to water in the mobile phase was 1:9, which resulted in longer peak elution time and lower yield.

In Comparative Example 1, the mobile phase was a mixture system of acetonitrile and water, a C18 column purification step was then required in the subsequent step, which led to severe radiolysis and increase in reaction time, and reduced the final yield and radiochemical purity.

In Comparative Example 2, since only ethanol was used in the mobile phase, impurities and the final product came out together. Sodium vitamin C and vitamin C are insoluble in ethanol, so the mobile phase could not contain sodium vitamin C and vitamin C, resulting in low purity and instability of the final Compound I product.

The experimental effect data of Examples 17-22 and Comparative Examples 3-8 are shown in Table 5.

**Table 5: Experimental effect data of Examples 17-22 and Comparative Examples 3-8**

| | Radiochemical purity at 0h (%) | 6h stability-radiochemical purity index (%) |
|---|---|---|
| Example 17 | 98.3 | 96.7 |
| Example 18 | 98.2 | 96.0 |
| Example 19 | 99.0 | 97.5 |
| Example 20 | 98.6 | 97.2 |
| Example 21 | 99.6 | 98.2 |
| Example 22 | 98.4 | 97.0 |
| Comparative example 3 | 82.1 | 64.6 |
| Comparative example 4 | 90.4 | 81.4 |
| Comparative example 5 | 83.5 | 73.6 |
| Comparative example 6 | 91.4 | 83.4 |
| Comparative example 7 | 91.8 | 84.0 |
| Comparative example 8 | 91.2 | 82.3 |

In Examples 17-22, the activity concentration of the product was 2000 MBq/mL, i.e., 54 mCi/mL. Examples 17 and 18 had high radiochemical purity and good stability for 6 hours.

In Example 19, the mass ratio of vitamin C to sodium vitamin C was 1:20, the radiochemical purity was high and the stability effect for 6 hours was good, and the sodium content was relatively high.

In Example 20 and Example 21, the ratio of vitamin C to sodium vitamin C was 2:5, and the radiochemical purity was high and the stability effect for 6 hours was good. In Example 21, the concentration of vitamin C was relatively high, so that the pH of the liquid composition of Compound I was relatively low.

In Example 22, when vitamin C was replaced with gentisic acid, the radiochemical purity was high and the stability for 6 hours was good, indicating that the gentisic acid in the composition played a role equivalent to that of vitamin C.

It can be seen from Comparative Examples 3-8 that when the liquid composition of Compound I did not comprise vitamin C or sodium vitamin C, or when vitamin C was replaced with L-glutathione, thiourea or sodium metabisulfite, the radiochemical purity was poor at 0h, the stability effect was even worse after 6h. For myocardial perfusion PET imaging agents, a radiochemical purity lower than 90% is unqualified.

This application provides a reagent kit for automated preparation of liquid composition of Compound I.

In some embodiments of the present application, the formula of reaction flask R1 is shown in Table 6.

**Table 6**

| Ingredient | amount per unit |
|---|---|
| Amino polyether | 15mg |
| Potassium carbonate | 1.5mg |
| Water for injection | 0.29mL |
| Acetonitrile | Adjust to 1 mL |

Preparation of potassium carbonate solution: weigh an appropriate amount of water for injection into a glass beaker, add the prescribed amount of potassium carbonate, rinse the container 2-3 times with a small amount of water before adding it to the beaker and stir to dissolve;
preparation of the eluent: add an appropriate amount of acetonitrile to a conical flask, add the prescribed amount of amino polyether, and rinse the container 2-3 times with a small amount of acetonitrile before adding to the conical flask and stir to dissolve; add the potassium carbonate solution prepared above and rinse the beaker 2-3 times with the remaining amount of water as per the prescription; then add it to the conical flask and stir well; finally, adjust the volume with acetonitrile and stir well;
filtration: the above eluent is filtered through two 0.22 µm sterilization-grade hydrophobic polytetrafluoroethylene capsule filters;
filling, plugging, and capping: the above filtered solution is distributed into 1mL aliquots per bottle, plugged, and capped;
lamp inspection and packaging: after passing the lamp inspection, the products are labeled and packaged in paper boxes with a specification of 1 piece/box, and then labeled, sealed and packed.

In some embodiments of the present application, the formula of reaction flask R2 is shown in Table 7.

**Table 7**

| Ingredient | | Amount | |
|---|---|---|---|
| | | mg | % |
| Acetonitrile solution of Compound I precursor (Note 1) | | Converted to 8 mg of compound I precursor | 0.4(w/v) |
| Acetonitrile | Adjusted to | 2mL | 99.6(v/v) |
| Note 1: amount of acetonitrile solution of Compound I precursor =8mg/ Concentration of Compound I Precursor Acetonitrile Solution [% (g/g)] | | | |

Preparation of Compound I precursor solution: weigh the prescribed amount of Compound I precursor acetonitrile solution with a beaker, transfer it to a wide-mouth blue cap bottle; rinse the beaker 2-3 times with a small amount of acetonitrile and combine them into the blue cap bottle; adjust the volume with acetonitrile and stir well;
filtration: the above Compound I precursor solution is filtered through two 0.22 µm sterilization-grade hydrophobic polytetrafluoroethylene capsule filters;
filling, plugging, and capping: the above filtered solution is distributed into 2mL aliquots per bottle, plugged, and capped;
lamp inspection and packaging: after passing the lamp inspection, the products are labeled and packaged in paper boxes with a specification of 1 piece/box, and then labeled, sealed and packed.

In some embodiments of the present application, the formula of reaction flask R3 is shown in Table 8.

**Table 8**

| Ingredient | Amount per unit (mL) |
|---|---|
| Anhydrous Ethanol | 10 |

Solution preparation: weigh the prescribed amount of anhydrous ethanol and add it to the solution preparation barrel; turn on the jacket cooling water to ensure the temperature inside the tank was below 20°C, and then stir well;
filtration: the solution in the preparation barrel is pre-filtered through a primary 5-inch 0.45 µm hydrophobic polytetrafluoroethylene filter, then filter through two secondary 5-inch 0.22 µm sterilization-grade hydrophobic polytetrafluoroethylene filters, and finally pressurize into the receiving tank;
filling, and plugging: the above filtered solution is distributed into 10mL aliquots per bottle, plugged, and capped;
lamp inspection and packaging: after passing the lamp inspection, the products are labeled and packaged in paper boxes with a specification of 1 piece/box, and then labeled, sealed and packed.

In some embodiments of the present application, the formula of prescription bottle P1 is shown in Table 9.

**Table 9**

| Ingredient | | Amount per unit | |
|---|---|---|---|
| | | g | % (w/w) |
| Polyethylene glycol 400 (for injection) | | 2.00 | 14.49 |
| Water for injection | Adjusted to | 13.8mL | NA |

Preparation of polyethylene glycol 400 solution: weigh the prescribed amount of polyethylene glycol 400 (for injection); add an appropriate amount of injectable water (<30°C) to the solution preparation barrel of polyethylene glycol 400, stir to dissolve for later use;
preparation of prescription matrix solution: add an appropriate amount of water to the preparation tank, transfer the polyethylene glycol 400 solution to the preparation tank, rinse the preparation barrel three times with an appropriate amount of injectable water (<30°C) and combine them into the preparation tank; stir well, and then quantify with injectable water (<30°C), stir well;
filtration: the solution is filtered through a primary 5-inch 0.45 µm hydrophilic polyethersulfone filter cartridge, then filter through a secondary 5-inch 0.22 µm sterilization-grade hydrophilic polyethersulfone filter cartridge, and finally pressurize into the receiving tank;
filling, and plugging: the above filtered solution is distributed into 13.8mL aliquots per bottle, plugged, and capped;
sterilization: the above capped intermediate product is sterilized under the following conditions: sterilization temperature is 121°C, sterilization time is 15 minutes, and F₀ ≥ 12;
lamp inspection and packaging: after passing the lamp inspection, the bottled products are labeled and packaged with plastic inner trays in paper boxes with a specification of 1 piece/box, and then labeled, sealed and packed.

In the present application, the prescription bottle P1 are used as a transit bottle, wherein the reagent in the prescription bottle P1 and part of the reagent obtained by dissolving another prescription bottle P2 in water together serve as the prescription matrix solution.

In some embodiments of the present application, the formula of prescription bottle P2 is shown in Table 10.

**Table 10**

| Ingredient | Amount per unit (g) |
|---|---|
| Vitamin C | 1.6 |
| Sodium vitamin C | 8.8 |

The formula of prescription bottle P2 is as follows:
weighing: weigh the prescription amount of vitamin C and sodium vitamin C;
aliquoting the powder, plugging and capping: use a high-precision dispensing machine for powder dispensing and capping, with a filing amount is 1.6g for vitamin C and 8.8g for sodium vitamin C; plug and cap them;
lamp inspection and packaging: after passing the lamp inspection, the bottled products are labeled and packaged with plastic inner trays in paper boxes with a specification of 1 piece/box, and then labeled, sealed and packed.

In this application, there are two prescription bottles P2. One can be used for the mobile phase, and the other can be used for storage of purified reagents.

Taking Example 1 as an example: before the reaction starts, the above prepared reaction flasks R1, R2, R3, and prescription bottle P1 are placed in their respective positions for reaction; after the completion of the ¹⁸F ion nucleophilic substitution reaction, high-performance liquid chromatography is performed for purification; the vitamin C and sodium vitamin C in the mobile phase are from prescription bottle P2; wherein reagents in the other prescription bottle P2 are dissolved in water to a specified volume in advance and a portion of the dissolved reagent is taken and mixed with the reagent from prescription bottle P1 to serve as the prescription matrix solution; after purification, Compound I is collected into the prescription bottle P1 that has been preloaded with the above prescription matrix solution to obtain a liquid composition of Compound I.

Although the present application has been disclosed as above by examples and embodiments, they are not intended to limit the present application. Any person having ordinary knowledge in the art may make some changes and modifications without departing from the spirit and scope of the present application. Therefore, the protection scope of the present application shall be determined by the scope of the patent application attached hereto.

## Claims

1. A liquid composition of Compound I, comprising one or more selected from the group consisting of vitamin C, sodium vitamin C, and gentisic acid;
the Compound I is 2-tert-butyl-4-chloro-5-((3-((4-((2-(2-fluoro[¹⁸F]ethoxy)ethoxy)methyl)-1*H*-1,2,3-tria zol-1-yl)methyl)benzyl)oxy)pyridazin-3(2*H*)-one.

2. The liquid composition according to claim 1, wherein the ratio of concentration of vitamin C (mg/mL) to activity concentration of Compound I (mCi/mL) is 0.02-0.2.

3. The liquid composition according to claim 1, wherein the ratio of concentration of gentisic acid (mg/mL) to activity concentration of Compound I (mCi/mL) is 0.02-0.2.

4. The liquid composition according to claim 1, wherein the ratio of concentration of sodium vitamin C (mg/mL) to activity concentration of Compound I (mCi/mL) is 0.04-1.

5. The liquid composition according to claim 1, wherein the sodium vitamin C is 2-10 parts by weight relative to 1 part by weight of the vitamin C.

6. The liquid composition according to claim 1, wherein the gentisic acid is 0.1-5 parts by weight relative to 1 part by weight of the vitamin C.

7. The liquid composition according to claim 1, wherein the liquid composition further comprises an aqueous solution of polyethylene glycol and/or ethanol.

8. The liquid composition according to claim 7, wherein the concentration of polyethylene glycol is 0.1-0.3 g/mL.

9. The liquid composition according to claim 7, wherein the ethanol is 5-20 mL relative to 100 mL of water.

10. A method for preparing the liquid composition according to any one of claims 1 to 9, wherein the method comprises: dissolving one or more of vitamin C, sodium vitamin C and gentisic acid in water and stirring to form a solution, and mixing Compound I collected with the solution to obtain the liquid composition of Compound I.

11. The method according to claim 10, wherein the method comprises: dissolving polyethylene glycols and/or ethanol, and one or more of vitamin C, sodium vitamin C and gentisic acid in water and stirring to form a solution, and mixing the Compound I collected with the solution to obtain the liquid composition of Compound I.

12. Use of the liquid composition of Compound I according to any one of claims 1 to 9 or the liquid composition of Compound I prepared by the method according to claim 10 or 11 in preparation of a myocardial perfusion PET imaging agent.

13. A method for performing myocardial perfusion on a subject, comprising administering to the subject the liquid composition of Compound I according to any one of claims 1-9, or the liquid composition of Compound I prepared by the method according to claim 10 or 11.

14. A reagent kit for automated preparation of a liquid composition of Compound I, wherein the reagent kit comprises a reaction flask and a prescription bottle;
the reaction flask includes reaction flask R1, reaction flask R2, and reaction flask R3; wherein the reaction flask R1 is used to hold the eluent for eluting ¹⁸F ion; the reaction flask R2 is used to hold a solution of Compound I precursor for the ¹⁸F ion nucleophilic substitution reaction; the reaction flask R3 is used to hold reagents for diluting the crude products and rinsing the reaction system;
the prescription bottle includes prescription bottle P1 and prescription bottle P2; wherein the prescription bottle P1 is used to transfer the reaction product of ¹⁸F ion nucleophilic substitution reaction; the prescription bottle P2 is used to hold substances used to stabilize the reaction product of ¹⁸F ion nucleophilic substitution reaction.

15. The reagent kit according to claim 14, wherein the reaction flask R1 comprises amino polyether, potassium carbonate, water for injection, and acetonitrile; preferably, the concentration of amino polyether is 5-40mg/mL, the concentration of potassium carbonate is 1.5-20mg/mL, and the volume ratio of acetonitrile to water is (0.25-19): 1;
alternatively, the reaction flask R2 comprises an acetonitrile solution of the Compound I precursor for ¹⁸F ion nucleophilic substitution reaction; preferably, the concentration of the Compound I precursor in the acetonitrile solution is 1-10 mg/mL;
alternatively, the reaction flask R3 comprises anhydrous ethanol.

16. The reagent kit according to claim 14, wherein the prescription bottle P1 comprises polyethylene glycol; preferably, the concentration of polyethylene glycol is 0.05-0.3 g/mL;
alternatively, the prescription bottle P2 comprises one or more of vitamin C, sodium vitamin C, and gentisic acid for stabilizing the reaction product of the ¹⁸F ion nucleophilic substitution reaction;
preferably, the concentration of vitamin C is 2-16mg/mL; the concentration of sodium vitamin C is 15-40mg/mL, and the concentration of gentisic acid is 2-16mg/mL.
